(19) Europäisches Patentamt
European Patent Office
Office européen des brevets

(11) **EP 1 847 831 A1**

(12) **EUROPEAN PATENT APPLICATION**

(43) Date of publication:
**24.10.2007 Bulletin 2007/43**

(51) Int Cl.:
*G01N 33/36* (2006.01)      *G01N 27/447* (2006.01)
*G01N 33/68* (2006.01)      *C07K 16/18* (2006.01)
*C12N 5/00* (2006.01)

(21) Application number: **07008034.6**

(22) Date of filing: **20.04.2007**

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**AL BA HR MK YU**

(30) Priority: **21.04.2006 IT GE20060047**

(71) Applicants:
• **Consiglio Nazionale delle Ricerche 00185 Roma (IT)**

• **CCMI - Cashmere & Camel Hair Manufacturers Institute Boston MA 02108 (US)**

(72) Inventors:
• **Patrone, Eligio 16146 Genova (IT)**
• **Vineis, Claudia 13897 Occhieppo Inderiore (BI) (IT)**

(74) Representative: **Pennacino, Enrico Porsia & Associati S.r.l. Via Brigata Liguria 3/16 A 16121 Genova (IT)**

(54) **Method for the determination of the composition of textile fibers**

(57) Method for the recognition and the quantitative evaluation of mixed textile fibers of animal source, comprising the steps of:
- preparing and conditioning of the fibers to be analysed;
- extracting of the proteins by means of demolition of the fibers, and subsequent centrifugation;
- dosing the mixture of proteins contained in the fibers;
- separing by electrophoresis the mixture of proteins;
- transferring the proteins on a membrane and reacting with at least a specific monoclonal antibody;
- marking with an enzyme conjugated secondary antibody; the said enzyme reacting with a substrate so as to provide a chemiluminescent product;
- detecting the chemiluminescence by means of a photoplate and;
- scanning and analysing of the obtained images by means of a processor provided with a suitable program.

Fig. 1

EP 1 847 831 A1

**Description**

The present invention relates to a method for the determination of the composition of the textile fibers, and particularly it relates to a method for the determination of the composition of mixed animal fiber, such as wool fibers of various animal source.

The analysis of mixtures of wool with other animal fibers, and particularly with cashmere, is presently performed in specialized laboratories, where the fibers are identified on the basis of their morphological features by means of optical microscopy or scanning electron microscopy (SEM). Very often, the morphological differences between fibers having different source are slight and difficultly appreciable; the diameter, the length, the shape and the dimensions of the cuticular cells, the presence and the shape of the pigments, are often of difficult interpretation. Moreover, some chemical and mechanical processing, such as dyeing, bleaching, raising, non-shrink treatments, give raise to modifications to the fibers such as to render the idenfication very difficult. Consequently, the proper recognition of the single fibers present in a textile material depends, in a large measure, on the experience of the operator; by this way it is evident the importance of the development of an alternative identifying method, so as to improve the objectivity and the reproducibility of the results.

Analytical ways based upon the analysis of the differences between the DNA chains in animal fibers have been tried out, however the results are not particularly impressing from the point of view of the univocality of the data obtained; moreover, these techniques show a number of problems related to the methods and the costs. Generally speaking, this approach gives some chance as a qualitative assay, but is not to be trusted from the quantitative point of view.

Recently, some studies have been performed about the use of immunological techniques for the identification of different animal fibers. The immunological technique shows the undeniable advantage of an highly specific response; however, the principal need of the wool manufacture industry is the availability of a liable method of quantitative determination, having reasonable operating time and costs, so as to allow its adoption as normal standard control.

An aim of the present invention is the finalization of a new objective analitical methodology, which exploits the utilisation of the immunological techniques, based upon the production and use of monoclonal antibodies able to recognize the species specific sequences of the primary structure of keratin, for identifying fibers from the different animal species.

An object of the present invention is therefore a method for the recognition and the quantitative evaluation of mixed textile fibers of animal source, comprising the steps of:

- preparing and conditioning of the fibers to be analysed;
- extracting the proteins by means of demolition of the fibers, and subsequent centrifugation;
- dosing the mixture of proteins contained in the fibers;
- separing by electrophoresis the mixture of proteins;
- transferring the proteins on a membrane and reacting with at least a specific monoclonal antibody;
- marking with an enzyme conjugated secondary antibody; the said enzyme reacting with a substrate so as to provide a chemiluminescent product;
- detecting the chemiluminescence by means of a photoplate and;;
- scanning and analysing of the obtained images by means of a processor provided with a suitable program.

In a preferred embodiment, the method comprises the preparation, for every sample having unknown composition, of an analytical set comprising at least a reference sample for each of the pure components of the mixture of fibers. However, the analytical set comprising at least a mixture at known titer, and preferably three mixtures at known titer, are considered as more liable; by this way it can be interpolation curve for the value relating to unknown sample.

Moreover, for each sample having unknown composition, it is preferred to perform the test using more than one different antibody, and particularly it is preferred to use three different antibodies. The use of more than one antibody confers an higher liability to the analytical assay.

Furthermore, it is preferred to couple the immunological assay with a reaction with a specific dyer for the proteic material, such as Coomassie Brilliant Blue R-250, so as to obtain a reference value for the image analysis which will be subsequently performed; the dyed gels are scanned as images so as the photographies of the membranes of the immunological test, and the response values of these latter will be therefore suitably normalized.

Another object of the present invention are specific monoclonal antibodies for the proteins of the fibers of animal source, and particularly for a certain class of proteins typical of the fiber for which the determination has to be effected. More particularly, the production of antibodies is performed from an antigen obtained by electrophoresys of the proteins resulting from the demolition of animal fibers of pure type; the said antigen comprises a specific and limited group of proteins of the said type of fibers.

Further advantages and features of the present invention will become more appearent from the following detailed description of an embodiment of the same, the said description being provided as a non limiting example.

*Preparation of the samples of animal fibers*

So as to carry out the analysis according to the present invention, either the fibers on which the analytical standard is based upon, that is to say the pure wool and cashmere fibers in the present case, or the fibers of the samples on which the evaluation has to be effected, have both to be prepared so as to used accordingly. Referring to the pure fibers, the preparation is also used so as to obtain the material useful for the production of the monoclonal antibodies, even if in this case the protein is submitted to an electrophoresys and a proteic fraction is choosen which is the most representative for such a fiber.

The destructive treatment conducted upon the fibers is in order to achieve the purification of the keratins composing the structure of the animal fibers. The said proteins have molecular weight comprised between 40000 ad 70000 u, and the composition of the said proteins is typical for several types of whool. Below is reported an example of the operations carried out for the preparation of both the standards, pure whool and cashmere; such an example can be similarly applied to samples for which the composition has to be detected.

*Materials*

§    About 150 mg of samples of pure cashmere and wool.
§    Soxhlet extractor
§    petroleum ether
§    Urea 8 M
§    Tris (Tris-hydroximethyl- aminomethane)-HCl 0,5 M
§    DTT (Dithiothreitol) 0,14 M
§    $Na_2EDTA$ 5 mM
§    5 mm filters
§    Centrifuge
§    -20°C freezer
§    Analytical balance
§    ventilated drying stove
§    BSA (bovine serum albumine).
§    DTT/Urea solution (Urea 8 M; Tris-HCl 0,5 M; DTT 0,14 M; $Na_2EDTA$ 5 mM)
§    concentrated dyer BIO-RAD Protein Assay (methanol and phosphoric acid)
§    Spectrophotometer.

*Methods*

*Fibers purification*

The samples are scoured and washed with petroleum ether in a Soxhlet extractor for two hours, with at least six cycles per hour. The petroleum ether is then evaporated from the sample and the same is dipped in water at room temperature; a dipping in water at 65°C will subsequently follow, stirring now and again, with a liquor ratio 1:100. The water excess is removed by squeezing, and the sample is placed under conditioning in standard atmosphere at 20°C and 65% R.H. for at least 24 hours.

*Protein extraction*

150 mg of fibers (conditioned at 20°C, 65% R.H. for at least 24 hours) have been cut in chips and placed in a test tube with 9,5 ml of a previously prepared reducing solution DTT/Urea (Urea 8 M, Tris-HCl 0,5 M, dithiothreitol 0,14 M, Na2EDTA 5 mM, pH 8,6) for four hours under nitrogen atmosphere.

The samples have to be filtered by means of 5 $\mu$m centrifuge filters and, after the elimination of the supernatant, the precipitate can be stored at-20°C.

*Protein dosing*

The protein concentration obtained by the extraction is determinated using the Bio-Rad method, that is to say using bovine serum albumine as a standard reference to perform a calibration curve as below reported.

- Calibration curve:

Preparation of a 200 μg/ml BSA solution (142,8 μl of 1,4 μg/ml BSA + 855,2 μl distilled water)

Preparation of the samples for the calibration as below reported in the table, by preparing two cuvettes for each point:

| BSA volume 200 μl/ml | Water volume | BSA final concentration |
|---|---|---|
| 0 μl | 800 μl | 0 μg/ml |
| 5 μl | 795 μl | 1 μg/ml |
| 15 μl | 785 μl | 3 μg/ml |
| 30 μl | 770 μl | 6 μg/ml |
| 50 μl | 750 μl | 10 μg/ml |
| 75 μl | 725 μl | 15 μg/ml |
| 100 μl | 700 μl | 20 μg/ml |

Addition to each cuvette of 1 μl of DTT/Urea solution (Urea 8 M, Tris-HCl 0,5 M, dithiothreitol 0,14 M, Na2EDTA 5 mM, pH 8,6) used for the protein extraction.
Addition of 200 μl of concetrated Bio-Rad dyer (BIO-RAD Protein Assay) and incubation for 40 min. providing that the indicated time will not be exceeded, so as to prevent the formation of precipitates which can interfere with the spectrophotometer analysis.

- Protein dosing of the samples:

Preparing two cuvettes for each sample with 800 μl of concetrated Bio-Rad dyer (BIO-RAD Protein Assay). Closing the cuvettes with parafilm ad agitating. Incubating for 40 min, providing that the indicated time will not be exceeded, so as to prevent the formation of precipitates which can interfere with the spectrophotometer analysis.
Performing the reading at the spectrophotometer at 595 nm after reset of the instrument air against air and then blank against blank (DTT/Urea solution).
From the values read for each point of the calibration, calculating the values A (intercept) and B (angular coefficient) of the best line passing for the given points (linear regression).

$$O.D. = A + B \; (\text{concentration } \mu g/\mu l)$$

$$(\text{concentration mg/ml}) = (\text{concentration } \mu g/\mu l) = (O.D. - A) / B$$

*Production of monoclonal antibodies*

*Preparation of the immunizing antigen*

The proteins of the pure fiber, in this specific case wool or cashmere, are extracted with the method as above described; 80 mg of total proteins dissolved in TUB + LYS are charged in a tube for isoelectrofocusing, and a run is effected for 3 days at 300 V. Subsequently, the electrophoresis is carried out for the second dimension on 10% SDS-PAGE gel. The proteins are then transferred on nitrocellulose for 3 hours and half. After a washing with water for five minutes, the substrate have to be dyed with R. Ponceau for five minutes, and the relevant band has to be cut. Thereafter, the membrane is washed with water until the complete discoloration is reached, and placed at -80°C for twenty minutes, without drying; then the menbrane is finely divided until a powder is obtained. The powder is recovered with 200 λ of saline solution, to which shall be added 200 λ of Freund adjuvant (complete only for the first immunization, incomplete for the next ones). For 80 μg of charged proteins are prepared 2-3 syrings with about 100 λ.

*Immunization*

The production of monoclonal antibodies was carried out from female Balb/c mice. The mice have been immunized with 4 intraperitoneal injections during a period of four months.

The primary immunization has been performed with 5-10 $\mu$g of Type II cashmere keratin in complete Freund adjuvant. The subsequent immunization is carried out with 5-10 $\mu$g of proteins in incomplete Freund adjuvant. Three days after the last treatment the mouse is sacrificed and the spleen is removed.

Lymphocytes are fused with mieloma P3/X63-Ag8 cells using 1 g/ml polyethilene glycol 4000 (Merck, Darmstadt, Germany). The reactivity of the supernatants of this cell culture in respect of cashmere and wool is evaluated by means of the dot blot technique.

The colonies showing a great difference in the immunoreaction with cashmere or wool keratins are cultured and analyzed using the Western Blot technique, on one or two dimensions.

The hybridoma cells showing an high immunoresponse are subcloned three times by means of the limit dilution technique.

*Quantitative production of monoclonal antibodies*

A number of techniques can be used so as to provide a large amount of monoclonal antibody. One of these techniques consists in expanding the hybridomas in vitro using suitable culture means; by this a concetration of antibody in the culture medium is achieved of about 5-50 $\mu$g/ml.

The cells are transferred in flasks with medium (Mega Cell RPMI 1640, Sigma) enriched with 3% FetalClone I (Hy Clone), both these products are able to allow a good cell growth even in conditions of low concentration of fetal bovine serum.

When the cells have reached a concentration of 5-7 x $10^5$ cells/ml, the culture medium is recovered, from which thereafter the monoclonal antibodies will be purified using the technique of ammonium sulphate precipitation.

*Precipitation of monoclonal antibodies with ammonium sulphate*

So as to perform a suitable purification of the immunoglobulins contained into the culture mediums of the hybridomas it is important to use well washed glass labware without any trace of soap, in order to prevent the blocking of the activity of ammonium sulphate. The 100% (SA) ammonium sulphate solution has to be prepared at least 24 hours before its use, so as to allow the best solubilization of the salt; the procedure is as follows: weighing 500 g of ammonium sulphate and transfering the same into a 500 ml flask; adding 100 ml of distilled water and then leave under stirring for some hours. After adjusting the solution to pH 7,00 with some drops of NaOH 1 M, adding distilled water until the volume of 500 ml is reached; then leave under stirring overnight at room temperature.

*Materials*

- SA solution (100% ammonium sulphate)
- SF solution (2% sodium azide, 1000 U/ml gabesate mesilate, Foy - Aventis). So as to prevent degradation and pollution of the monoclonal antibodies, 1 ml of this solution has to be added every 100 ml of supernatant; the final concentration will be 0.02 % sodium azide and 10 U/ml Foy.
- PBS
- Centrifuge
- Stirrer
- Dialysis tubes

*Method*

- Dialyze 450 ml of supernatant against PBS + SF for at least 24 hours at 4°C.
- Place the supernatant in a clean container with a hook under gentle stirring and add dropwise 130 ml of SA (22% final concentration). Adjust to pH 6,00 with HCl 1N.
- Leave under stirring for 30 min. at 4°C.
- Centrifuge at 3000 g for 30 min. at 20°C.

- Transfer the supernatant in a container with a hook under gentle stirring and add dropwise 270 ml of SA (47% final concentration). Adjust to pH 6,00 with HCl 1N.
- Leave under stirring at 4°C overnight.
- Centrifuge at 12000 g for 30 min. at 20°C.
- Recover the pellet with 5-10 ml of PBS + SF and transfer it into dialysis tubes.
- Apportion in aliquots of 0,5 - 1 ml and store at -20°C. Don't freeze and unfreeze repeatedly.
- Proceed to titration of the purified antibody by means of Dot Blot with decreasing concentrations of monoclonal antibody (concentration range to be evaluated: 1:10 to 1:100).

*Dot Blot*

This technique is used to perform the first screening of the clones obtained after the fusion, in order to chose the clones during subcloning of an hybridoma line and in order to define the concentration at which the monoclonal antibody, obtained after the precipitation with ammonium sulphate, has to be used.

2 $\lambda$ of the antigen solution (10 $\mu$l of wool or cashmere keratins + 80 $\mu$l of Sample Buffer) are directly applied on each square having 1 cm of side length of a nitrocellulose filter, which has been previously washed with TBST (Tris Buffered saline + 0,1% Tween 20). The nitrocellulose is placed in 3% milk solution for 30 min. and then washed with TBST. 2$\lambda$ of the culture medium of the hybridomas that has to be assayed are placed on each filter. After 15 min. the filter is washed three times with TBST and reacted with a secondary antibody conjugated with an enzyme (peroxydase) for 30 min. After a few washings with TBST, the chemiluminescence reaction of the conjugated enzyme is carried out. The results thus obtained provide the datum relating to the most effective clone or to the best concentration of antibody that has tobe used for the western blot analysis.

*Polyacrylamide gel electrophoresis in presence of SDS*

Polyacrylamide gels in presence of SDS are prepared according to a well known method, which is not therefore further herein described; it has to be noted that the gel preparation has to be effected 12-18 hours before the beginning of the electrophoretic scan.

The proteins that have to be analyzed are diluted into the sample buffer so as to obtain a concentration of 2 mg/ml. The diluted samples are denatured by immersing them in water at 90°C for 3 min. In order to have an amount of 30 mg of total protein for each well, the samples are in introduced in the wells using an Hamilton microsyring (9 to 30 ml maximum for each well). In the last free well is introduced bromophenol blue, which is useful as marker for controlling the end of the electrophoretic scan. The electrophoresis is carried out at a temperature of about 11°C. The power supply is programmed as follows:

1st step: 300 V, 60mA, 1 hour;
2nd step: 30 V, 5 mA, 15-16 hours;
3rd step: 300 V, 60 mA, 1 hour.

The values as referred to above relate to an electrophoresis in which two gels are used.

When the marker reaches the bottom of the gel, the electrophoresis is ended. The gels can then be cut and used in the next steps.

*Staining with Coomassie brilliant blue*

At the end of the electrophoresis, a gel is dipped in the dyer solution (0,1% Coomassie, 45% methanol, 10% acetic acid) for at least two hours under continuous stirring at room temperature.

Subsequently, the dyer is eliminated and the decolorizer is added (20% methanol, 75% acetic acid), always under stirring, changing several times the said solution until the strips result well contrasty in respect of the background of the gel. The said gel will be used as a control in order to verify that the concentration of the charged samples is uniform.

*Western Blot*

*Materials*

- Tris (tris-hydroxymethyl-aminoethane)

- Glycine
- SDS (sodium dodecylsulphate)
- Methanol
- NaCl
- Distilled water
- TWEEN 20
- Microbiologically pure dry milk
- Primary antibody
- Peroxidase-conjugated secondary antibody
- PVDF membrane
- ECL plus western blotting detection system -Amersham containing solution A and solution B
- Hyperfilm™ ECL plates-Amersham
- Kodak fixer and replenisher - Sigma
- Kodak developer and replenisher- Sigma
- Blot sponges
- 3M paper
- Colloidal Gold Total Protein Stain - Bio-rad
- Exposure box for 24 x 30 cm plates, metal bar closure- Sigma Solutions:

- transfer buffer: 48 mM Tris, 39 mM glycine, 0,1% SDS, 20% methanol, pH 8,7-8,9
- TBST 0.1% : 10 mM Tris, 0.15 M NaCl, 0.1% TWEEN 20, pH 7.4
- TBST 0.5% : 10 mM Tris, 0.15 M NaCl, 0.5% TWEEN 20, pH 7.4
- Milk 3%: 10 mM Tris, 0.15 M NaCl, 0.5% TWEEN 20, 3% milk, pH 7.4
- Milk 3% + primary antibody (the dilution depends on the initial concentration of the same antibody)
- Milk 3% + secondary antibody (diluted 1:1000)

*Metodo*

The proteins are transferred on PVDF membrane; the transfer is carried out by forming a sandwich comprising, from the anode, a sponge, a 3M paper sheet, PVDF membrane, gel, a 3M paper sheet, a second sponge. The sandwich is immersed in a thermostatic bath of transfer buffer and connected to power supply at 60 V for three hours. At the end of the procedure the gel can be stained with Coomassie, according to the method as above described, so as to carry out the control upon the transfer of the proteins onto the membrane.

The membrane is saturated with 3% for one hour and half at 4°C under constant stirring. The milk is then substituted with the primary antibody suitably diluted in 3% milk and leaved under stirring at 4°C overnight.

The following day three washings are carried out of 15 min. each with TBST 0,1%, always at 4°C and under stirring. At this stage the secondary antibody in introduced, diluted in 3% milk, under constant stirring for one hour and half at 4°C. Thereafter, three consecutive washings of 15 min each are performed with TBST 0,1%, and two other washings for 15 min. each with TBST 0,5%. Then the membrane is placed on a folded plastic transparent sheet, and 1,5 ml of solutions A and B previously mixed (1,5 ml of solution A and 25 ml of solution B, dose for each membrane) are poured on the membrane and allowed to react for 5 min.

Subsequently, the excess of ECL is eliminated, the plastic sheet is dried, and the membrane is gently displaced and positioned again in the initial stance; the sheet is folded again, the bubble that eventually have been formed between menbrane and plastic are removed, and sheet is placed in the exposure box.

*Development and fixing of the plates*

A plate is positioned above the membrane, the box is then closed for the exposure and the exposure times are timed.

After the desired exposure time, the plate is picked up and introduced in the developing tank for some minutes and fluttered; thereafter dipped in the fixing tank until the same plate becomes transparent. The plate is washed in current water for some minutes, and then dried. The exposure can be repeated several times with more longer exposure times. The exposure time of 30 min. has not to be exceeded, because after the said time the ECL decays. The membranes, after the development, are firstly washed for about 30 min, with water under constant stirring, then are stained with Colloidal Gold, which imparts a brown color to the proteic strips; by this way it is possible to highlight the transfer of the proteins onto the membrane.

*Image analysis*

The identification of the cashmere percentage in unknown samples comprises the preparation of two monodimensional gels in which have to be charged, toghether with the unknown samples, a sample at 100% cashmere and one at 100% wool, the said samples will be used for the definition of a calibration curve. A gel will be stained with Coomassie brilliant blue, in order to determinate the concentration of the samples, whilst the second sample will be used in the Western Blot's experiments with monoclonal antibodies specific for cashmere type II keratins.

Using the computer program Quantity One (BioRad) for the quantitative analysis of monodimensional gels and plates, the experimental results as below reported are evaluated.

The acquisition of the images is carried out with a densitometric scanner GS-800 having a resolution of 63 mm both on X and Y axis, by using a a green filter for the plates and a red filter for the Coomassie-stained gels. The images thus obtained are optimized using a median filter having a 3x3 matrix. For each experiment are analysed both the images of the Coomassie brilliant blue-stained gel and of the plates after ECL relating to the different monoclonal antibodies used in Western Blot.

The subsequent steps comprise the segmentation, with which are obtained binary images containing the objects that have been selected for the measurement and in the measurement of the parameter interesting the selected objects; the said parameter is the integrated optical density (IOD), which is defined as the sum of the individual OD of every pixel in the measured area:

$$IOD = \sum OD \cdot Area$$

The use of only two calibration points works well for the determinations which does not imply neither aggregation nor degradation of cytokeratins. Three calibration points for each pure component suffice in order to obtain accurate determinations. Generally speaking, a first evaluation can be achieved by using only the values of optical density not having been normalized; however, the use of the normalized optical density is always preferable, so as to avoid errors due to an inaccurate dosing with Bio-Rad protein assay.

Because the plate works according to the principles of a normal negative, the development conditions have to be controlled with great accuracy. A critical factor is represented by the concentration of the sample, that has to be kept around about 30 μg; concentrations very different from this latter can lead to under or overexposure of the plate. Moreover, it is appropriate to perform a preliminary calibration, by determining the optical density in function of the exposure time; the curve related to the photographic emulsions has a sigmoid trend and then the couple exposure time against concentration has to be fitted in the linear range. As discussed above, a last critical factor is represented by the concentration of the antibody that has to be determined every time that a new batch is used, or when the response shows a sensible variation depending on time.

The method according to the present invention will appear with greater evidence in view of the non limiting example as below reported; the said example refers to the appended tables of drawings, in which:

Figure 1 is a schematic plot of optical density against elution factor relating to the example 1;

Figures 2A and 2B are two plots showing the correlation between normalized optical density and concentration of the sample; and

Figure 3A and 3B are images respectively of the gels dyed with Coomassie and of the exposed plate after the immulogical assay of the samples.

## Example 1

So as to verify the correlation between the reactivity of a given antibody and the concentration of the different kind of fiber, five samples have been prepared at known concentration of fibers of cashmere and wool, and designated as below:

- sample 1: 100% cashmere
- sample 2: 70% cashmere; 30% wool
- sample 3: 50% cashmere; 50% wool
- sample 4: 30% cashmere; 70% wool
- sample 5: 100% wool.

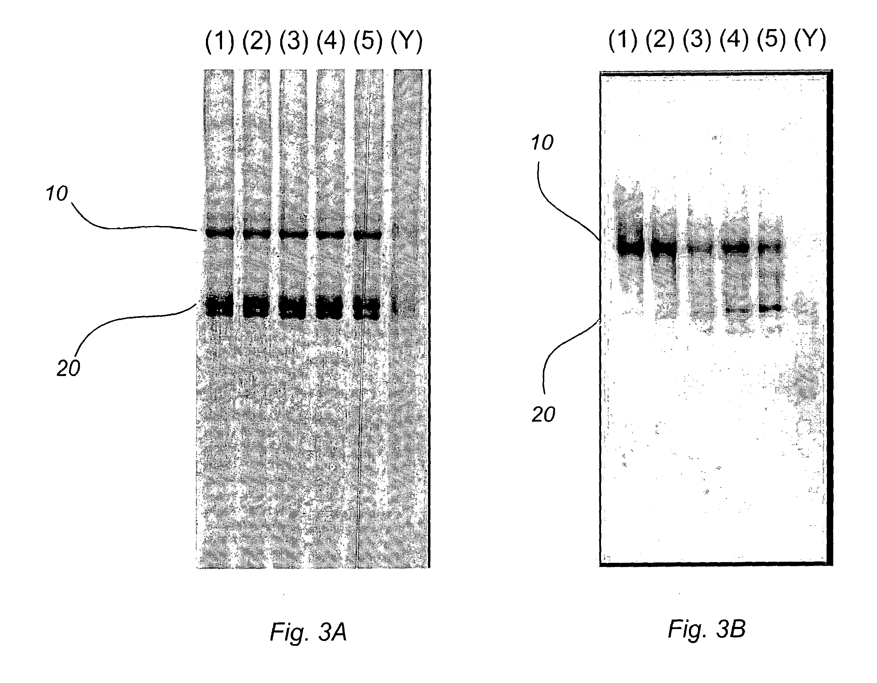

The samples are subjected to the assay according to the method of the present invention, taking the different operational steps as above referred. The protein fraction that has been analysed is that comprising the keratins which are conventionally designated as K8 and K18. In Figs. 3A and 3B are respectively reported the images obtained after the staining of the proteins on the electrophoresis gel (Fig. 3A) and after the exposure of the plate following the western blot analysis which in turn follows the reaction with the antibody (Fig. 3B). In both the figures appears a gel strip indicated with Y; the said test relates to a sample of yak hair, which however does not show proteins reacting with the specific antibody used herein.

As it can be noted, two zones relating the different keratins are well visible on the gels, respectively indicated with 10 for K8 and 20 for K18. It is peculiar the fact that the antibody, as it results from Fig. 3B, appears to be much more reactive with K8 of cashmere and K18 of wool.

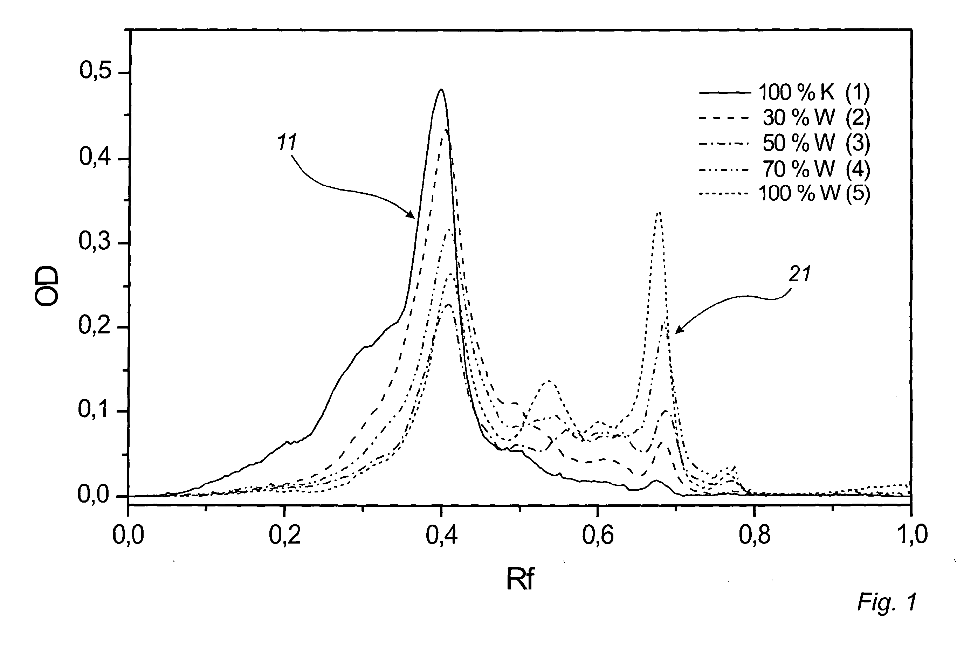

The plot of Fig. 1 shows the optical density curves in function of the elution factor for the different samples analysed; the peaks relating to the keratins K8 and K18 are respectively indicated with 11 and 21. As it can be noted, the trend reflects with fidelity the results of the analysis of the photographs previously described.

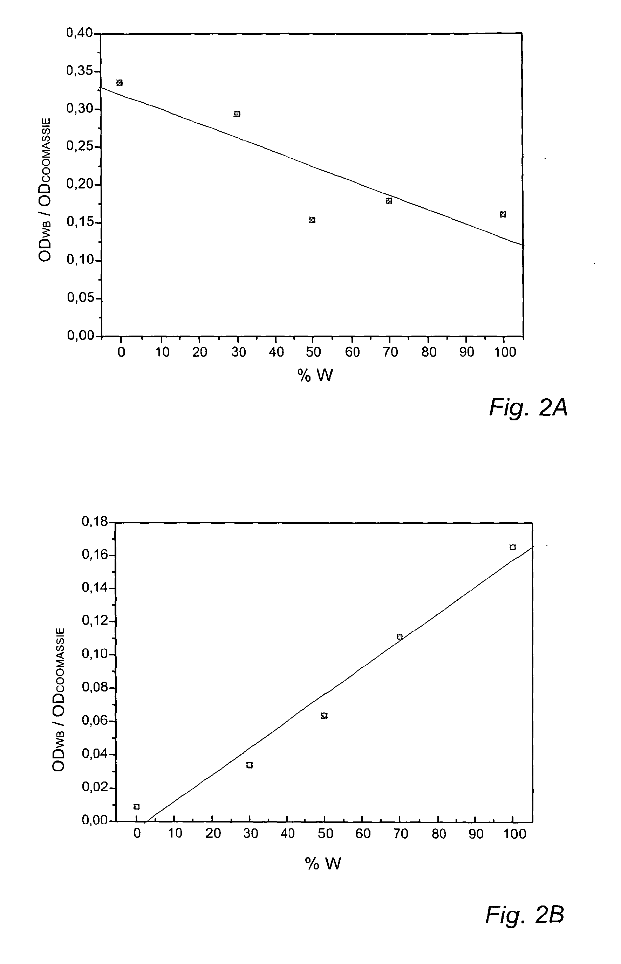

The plot of the fig. 2A shows, referring to the protein K8, the correlation between the concetration of wool fiber in the original sample and the normalized optical density, that is to say obtained by dividing the value of the optical density detected by the western blot analysis with the value obtained with the Coomassie staining. The linearity of the response seems quite satisfactory. Undoubtedly, much more convincing is that what results from the Fig. 2B, in which is provided the same correlation as above, this time relating the protein K18; the correlation between the data is in this case very near to the complete linearity.

## Claims

1. Method for the recognition and the quantitative evaluation of mixed textile fibers of animal source, comprising the steps of:

    - preparing and conditioning of the fibers to be analysed;
    - extracting of the proteins by means of demolition of the fibers, and subsequent centrifugation;
    - dosing the mixture of proteins contained in the fibers;
    - separing by electrophoresis the mixture of proteins;
    - transferring the proteins on a membrane and reacting with at least a specific monoclonal antibody;
    - marking with an enzyme conjugated secondary antibody; the said enzyme reacting with a substrate so as to provide a chemiluminescent product;
    - detecting the chemiluminescence by means of a photoplate and;
    - scanning and analysing of the obtained images by means of a processor provided with a suitable program.

2. Method according to the claim 1, comprising the preparation, for every sample having unknown composition, of an analytical set comprising at least a reference sample for each of the pure components of the mixture of fibers.

3. Method according to the claim 2, in which the analytical set comprises at least a mixture at known titer.

4. Method according to the claim 2, in which the analytical set comprises at least three mixtures at known titer.

5. Method according to anyone of the preceeding claims from 2 to 4, in which the reaction with the proteins comprise more than one different antibody, and particularly three different monoclonal antibodies.

6. Method according to anyone of the preceeding claims from 1 to 5, in which, after the separation by electrophoresis, a reaction is carried out on a portion of the proteins extracted from the fibers with a specific dyer for proteic material.

7. Method according to the claim 6, in which the said dyer is Coomassie Brilliant Blue R-250.

8. Method according to the claim 6 or 7, in which the said portion of proteins which has been stained is scanned as an image with suitable means, the said image being suitably processed so as to normalize the image scanned from the photoplates obtained from the immunological assay.

9. Method according to anyone of the preceeding claims from 1 to 8, in which the said step of preparing and conditioning of the fibers comprise: scouring and washing the fibers with petroleum ether in a Soxhlet extractor for two hours, with at least six cycles per hour; evaporating the petroleum ether from the sample; dipping the sample in water at room temperature; a dipping in water at 65°C will subsequently follow, stirring now and again, with a liquor ratio 1:

100; removal of water excess by squeezing, and placing the sample under conditioning in standard atmosphere at 20°C and 65% R.H. for at least 24 hours.

10. Method according to anyone of the preceeding claims from 1 to 9, in which the said extraction of the proteins is carried out by reacting the fibers with a solution of dithiothreitol and urea for four hours under nitrogen atmosphere.

11. Method according to claim 10, in which the said solution comprises: Urea 8 M, Tris-HCl 0,5 M, dithiothreitol 0,14 M, Na2EDTA 5 mM, at pH 8,6.

12. Method according to anyone of the preceeding claims from 1 to 11, in which the dosing is carried out by means of BIO-RAD Protein Assay.

13. Monoclonal antibodies specific for the proteins of fibers of animal source.

14. Monoclonal antibodies according to claim 13, **characterized by** the fact that the said antibodies are specific for the fibers of sheep wool.

15. Monoclonal antibodies according to claim 13, **characterized by** the fact that the said antibodies are specific for the fibers of cashmere wool.

16. Antibodies according to the claims 14 or 15, **characterized by** the fact that the said antibodies are specific for certain keratins which are present in the fibers of the sheep wool and/or the cashmere wool.

17. Hybridized cell lines for the production of specific antibodies for the fibers of animal source.

18. Cell lines according to the claim 17, obtained by fusion of limphocytes of mice Balb/c, immunized with proteins of fibers of animal source, with mieloma P3/X63-Ag8 cells and subsequent subcloning by limit dilution.

Fig. 1

Fig. 2A

Fig. 2B

(1) (2) (3) (4) (5) (Y)

10

20

Fig. 3A

(1) (2) (3) (4) (5) (Y)

10

20

Fig. 3B

EP 1 847 831 A1

**European Patent Office**

**EUROPEAN SEARCH REPORT**

Application Number

EP 07 00 8034

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | PALUZZI, S. ET AL.: "Anti-keratin Monoclonal Antibodies for Identifying Animal Hair Fibers" TEXTILE RESEARCH JOURNAL, vol. 74, no. 5, 2004, pages 458-464, XP002448425 * the whole document * | 1-18 | INV. G01N33/36 G01N27/447 G01N33/68 C07K16/18 C12N5/00 |
| X | FRENCH P W ET AL: "Localization of low-sulfur keratin proteins in the wool follicle using monoclonal antibodies." THE JOURNAL OF CELL BIOLOGY APR 1986, vol. 102, no. 4, April 1986 (1986-04), pages 1412-1418, XP002448426 ISSN: 0021-9525 * the whole document * | 13-18 | |
| A | PURVIS IAN WILLIAM ET AL: "Major genes and QTL influencing wool production and quality: a review." GENETICS, SELECTION, EVOLUTION. : GSE 2005, vol. 37 Suppl 1, 2005, pages S97-107, XP002448427 ISSN: 0999-193X * the whole document * | 1-18 | TECHNICAL FIELDS SEARCHED (IPC) G01N C07K C12N |
| A | PLOWMAN JEFFREY E ET AL: "Problems associated with the identification of proteins in homologous families: the wool keratin family as a case study." ANALYTICAL BIOCHEMISTRY 15 JAN 2002, vol. 300, no. 2, 15 January 2002 (2002-01-15), pages 221-229, XP002448428 ISSN: 0003-2697 * the whole document * | 1-18 | |

The present search report has been drawn up for all claims

| Place of search | Date of completion of the search | Examiner |
|---|---|---|
| The Hague | 3 September 2007 | VON EGGELKRAUT, R |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document